# EUROPEAN PATENT SPECIFICATION

(11) **EP 1 965 325 B1**
(45) Date of publication and mention of the grant of the patent: **09.05.2018**
(21) Application number: 07425110.9
(22) Date of filing: 01.03.2007
(51) Int. Cl.: G06F 19/00

(54) **Therapeutic-diagnostic device**
Therapeutische Vorrichtung
Dispositif thérapeutique

(43) Date of publication of application: 03.09.2008
(73) Proprietor: BRACCO IMAGING S.p.A., 20134 Milano (IT)
(72) Inventor: Canepa, Valtero, Bioggio (Svizzera) (IT)
(74) Representative: Karaghiosoff, Giorgio Alessandro

(56) References cited:
- WO-A-2006/083933
- WO-A-2006/108026
- WO-A-2006/129301
- US-A1- 2002 038 392
- AIM WHITE PAPER: "Introduction to Radio Frequency Identification (RFID) - A Basic Primer" INTERNET CITATION, [Online] 23 August 2001 (2001-08-23), XP002372243 Retrieved from the Internet: URL:http://www.aimglobal.org/technologies/ rfid/resources/RFIDPrimer.pdf> [retrieved on 2006-03-15]

## Description

The present invention relates to a therapeutic/diagnostic network, according to the preamble of claim 1

At present it is known the fact of associating electronic identification tags to drugs for example of the type called RFID or the like, in order to have more information about the drug and to better manage storehouses and the production. Drugs that are univocally associated to the RFID tag allow also to be provided with a certificate of originality of the drug and moreover allow information for using it to be associated to the drug and to be available.

These drugs in said containers associated with RFID tags are used in combination with dispensing devices provided with means communicating with identification devices and providing to display information.

For example it is known a drug dispensing device, so called intelligent syringe, provided with a unit communicating with the RFID tag of the drug container and it retrieves and displays information about the originality and the use protocol on a viewer, allowing operating personnel to identify the drug validity and to set suggested or prescribed dispensing modalities and dosage.

However, at present, the RFID tag is used only for recognizing the drug originality and for said task
providing information. Moreover possible information about the use, that is dosage and dispensing protocols are provided only as passive information that can be used by the user. Therefore as regards the use of the drug-container unit comprising the drug, the container and the RFID tag it is limited to the mere drug-container unit and dispensing device combination with an interacting modality that is very primitive and limited as regards functional point of view.

WO2006083933 discloses a system for automatically delivering an infusate to a patient including an infusion set and an infusion device. A signaling component disposed on an infusion set component identifies an administration protocol for the infusion set. A detection device operatively connected to the infusion device detects the signaling component and identifies the administration protocol. The infusion device is then configured to operate according to the administration protocol.

US2002038392 discloses a method and an apparatus for controlling and monitoring medication delivery. Information tags on the bags are provided that specify delivery parameters. The delivery parameters are obtained from at least one bag. A controller is associated with a patient. Patient information for the particular patient is compared with the delivery parameters, and the efficacy of delivering the medicant to the patient is determined. A delivery pump is controlled as a function of the comparison according to
various timing rules and other verification procedures.

WO2006129301 discloses an apparatus for use with at least one labeled radiopharmaceutical agent. The apparatus includes a container containing the labeled radiopharmaceutical agent and a portable data carrier associated with the container. The data carrier containing imaging protocol information for use with the at least one labeled radiopharmaceutical agent and is able to communicate with a computer.

WO2006108026 discloses a radiopharmaceutical administration device supporting a radiopharmaceutical container having an RF data tag for use in a radiopharmaceutical administration procedure. The radiopharmaceutical administration device comprising an electromagnetic device for reading data from the data tag. The data comprising one or more of: the amount of a radiopharmaceutical in the radiopharmaceutical container, the identity of a radiopharmaceutical in the container, manufacturing information for the radiopharmaceutical in the container, the radioactivity level of the radiopharmaceutical in the container, a use code for the radiopharmaceutical in the container, configuration information for a radiopharmaceutical administration device used with the container, and a particular radiopharmaceutical container previously used with the radiopharmaceutical administration device.

The invention aims at providing a therapeutic/diagnostic device wherein the univocal drug
and identification device combination, particularly the drug-RFID tag one, is a drug-container operating unit and it acts like an active element being part of a plurality of operating elements or tools intended to perform different tasks that can be provided in a diagnostic and/or therapeutic device. Such operating modality of the drug-container unit is substantially equal to the one of a mechanical member or of an operating unit in a complex system or machine. Therefore the invention aims at overcoming the current concept of drug and its use modalities as a medium separated from devices intended to carry out diagnostic and/or therapeutic operations.

The invention aims also at providing a drug-container operating unit that in combination with other operating units overcomes the mere and conventional concept of operating unit being integrated with other operating units in a therapeutic/diagnostic system.

The invention achieves the above aims by means of a therapeutic/diagnostic network according to the preamble of claim 1 further comprising the combination of features of the characterizing part of claim 1.

Particularly, the operating unit is composed of an hardware/software system comprising a control unit and one or more peripherals intended for carrying out predetermined tasks and which peripherals are controlled by the control unit by a general control software, while specifications for operating said peripherals are contained in administration and dispensing protocols that therefore are equal to or can be considered equal to drivers of computer peripherals or the like.

Said operating unit can be of the automatic or semi-automatic type and can be composed of a computer comprising a central processing and control unit and a plurality of peripheral units, a general program for controlling individual peripherals being stored in the central processing and control unit, while the drug-identification device unit is a further peripheral that automatically provides drivers for using it comprising information for controlling further peripherals according to operating protocols provided for the drug.

Particularly an embodiment provides two or more different administration and dispensing protocols or two or more different use protocols for the drug for two or more different use conditions to be stored in the memory of the identification device associated to the drug a peripheral for displaying and selecting the protocol to be used being provided, which selection is carried out manually or by auomatic means and it causes the selected protocol to be loaded in means controlling the operating unit.

An embodiment of the invention provides means controlling the operating unit to read the memory of identification devices associated to drugs and to display use protocols, while the operating unit is provided with at least a displaying screen and with at least means for selecting and inputting commands, such as for example a monitor and a mouse or other similar means.

With reference to the above it is clear that the the drug unit and local unit combination according to the present invention is limited to the combination itself even if the task of the drug unit and of the local unit are deeply different with respect to conventionally known ones.

However, by means of the above inventive teaching, the new concept of drug consisting in said drug container unit wherein the term container means both the drug container or the packaging thereof and the identification device, allows to go beyond the concept of drug operating unit in the conventional meaning of the operating unit term and so it allows to make a diagnostic/therapeutic system particularly a network. In such system or network, the drug directly interacts, as an operating unit, with two or more operating units, such as for example a drug dispensing unit, with one or more diagnostic machines and/or machines for carrying out therapeutic tasks. This allows a direct interaction among operating units used when performing a diagnostic/therapeutic operation.

Therefore a further improvement of the present invention provides the therapeutic/diagnostic device to be as a diagnostic or therapeutic network and to comprise the drug container or various containers each one for a different drug or the packaging of drug or drugs an identification device is associated to each one of such containers, the drug with the identification device, as an operating unit, being in communication in a direct or indirect way with two or more further operating units having different tasks and being intended to be used for carrying out a diagnostic/therapeutic operation.

In the simplest arrangement said diagnostic/therapeutic network can be composed of a drug-identification device unit, a drug dispensing unit and at least a further local operating unit or also two or more different local operating units and intended to carry out different operations on with or in the presence of the drug.

One or more of said local operating units can read protocols for administrating, dispensing and using the drug in a direct way and/or by a further central control unit intended to act in combination with the first one. In this case said protocols contain specific information for controlling all or at least a part of the local operating units intended to act in combination one with the other.

A configuration example of a diagnostic/therapeutic network provides one of the operating unit to be composed of a drug dispenser that can be of the automatic or semi-automatic type, and/or a diagnostic apparatus and/or an apparatus for acquiring diagnostic images and/or an apparatus for carrying out therapeutic operations.

Such configuration can provide the drug to be constituted by a contrast medium.

A more detailed constructional form of a diagnostic/therapeutic network of said example provide a first operating unit to be intended for delivering contrast media in an automatized way, such as the injection or consumption thereof by the user at predetermined amounts and also with predetermined dispensing time or dispensing steps according to the kind of test and/or the kind or anatomical region or organ. A second operating unit is composed of at least an image acquisition machine, which acquisition is syncronized and provides predetermined acquisition steps as well as predetermined acquisition setting parameters and that are specifically set for the kind of test and/or anatomical region and organ. Data for administrating, dispensing the drug and for performing diagnostic test are provided and taken from administration, dispensing protocols and from protocols for performing the diagnostic test that are contained in the memory of the drug identification device or however are provided by said identification device associated to the drug. Said operating unit can be provided with means for displaying said protocols and with means for selecting one of said protocols by the user and therefore with means transmitting and loading individual protocols in different operating units and i.e. in the drug dispensing unit and in the unit performing the diagnostic test as specialized control drivers.

As a further improvement the invention provides means for pre-setting the modality for using the drug such as the anatomical region or organ wherein the drug has to be dispensed, and the type of diagnostic test. These information can be directly set in the control unit of one of the local operating units and/or of all local operating units said setting of how to use the drug being used as a automatic control selecting the specific protocol for administrating and dispensing and using the drug for different local operating units provided for performing the pre-set diagnostic test.

Above mentioned operations can be completely or at least partially automatized, by providing means for detecting settings operating different local operating units providing said settings to comparing means. Comparators compare them with specific settings defined in the protocol for using the drug and stored in the identification device, which comparing means generate an alarm signal or automatically change settings for operating the operating unit or units when said comparison detects a difference between settings defined in protocols for administrating, dispensing and using the drug and operating settings set in the local operating unit or units.

An embodiment variant provides a central control unit composing the centralized receiving/transmitting station and communicating both with the drug identification device and with one or more or all provided local operating units. Said centralized receiving/transmitting station exchanges information, that is parameters for administrating, dispensing and using the drug provided in protocols stored in the identification device with different local operating units. Said centralized receiving/transmitting station possibly comprises said comparing means, said warning means and said means automatically changing settings of operating units.

Advantageously still according to a further improvement of the device according to the present invention the network has a structure of the client-server type or it is a network with a peer-to-peer architecture.

An advantageous example of the network structure provides the diagnostic/therapeutic network to comprise a central unit managing information about each patient comprising means for the communication with the drug identification device and/or with one or more operating units in a direct way or by means of other local communication units.

In an advantageous embodiment, said central management unit comprises personal databank of patients wherein diagnostic and therapeutic information and possible contraindication conditions with reference to specific drugs are associated to each patient, said information being used as a means for automatically selecting the diagnostic test and/or the therapeutic action to be carried out and so as a means also for automatically selecting protocols for administrating and dispensing the drug and setting protocols for operating local operating units necessary to carry out diagnostic or therapeutic operations.

Particularly, but not exclusively, when an embodiment of the network of the type described above is provided, it is advantageous for the local operating unit or units dispensing the drug and performing operations to transmit dispensing and operating setting protocols used in performed operations to the central unit managing the patient database which protocols are stored in the patient database together with performed operations.

In this case an improvement of the invention provides the presence of a further operating unit managing a drug database comprising a memory wherein a drug database is stored and corresponding administration, dispensing and use protocols, a central unit having access to data of said database and means for the communication of said operating unit managing the drug database with one or more of the further operating units and with the drug identification device.

The efficacy of the network or system is guaranteed by a logic program controlling the functional interaction between the drug, the device identifying it, drug dispensing means and/or possible one or more further operating units. This logic program can be executed by the operating units or by a central control unit and said logic program provides the drug identification device to provide the drug identification code. The central unit managing the patient database provides the kind of operation to be carried out and characteristics of the patient, as well as possibly the kind of operating units provided for performing the operation to be carried out. Said information are verified to be sent to the operating unit managing the drug database that in turn provides to the operating unit dispensing the drug and to different operating units, protocols for administrating, dispensing and using the drug and protocols for setting and controlling different operating units provided for performing diagnostic and/or therpaueitc operations in combination with said drug respectively in a direct way or by a central control unit.

Further improvements and advantageous characteristics of the invention are object of subclaims.

From the above advantages deriving from the device according to the present invention are clear, both in the case of a simple combination of the drug together with the identification device with an operating unit, and in the case of a diagnostic/therapeutic network.

The high flexibility and also the possibility of optimizing time costs and above all modalities for dispensing and using the drug and modalities for performing diagnostic and/or therpeutic operations under the presence of said drug are not the only advantages. The fact of obtaining a network comprising also a patient database and a drug database managed also by different bodies or companies guarantees the greatest safety and correctness of use protocols. The diagnostic/therapeutic network allows also to have a fast and constant integration of clinical information under various conditions for using the drug both as regards patient characteristics, and as regards diseases and evolution degrees thereof and also as regards side effects or interactions with other drugs or with pathologic conditions of the patient.

Therefore the certainty of a constant update of the drug effects under various conditions is maximized guaranteeing the greatest trasparency and the greatest accessability of clinical data by pharmaceutical companies and medical organizations. The latter will be able to provide in real time clinical information to pharmaceutical companies that will be able to make information available for all drug users by means of the drug database.

According to a further improvement, the device according to the present invention provides a further operating unit composed of a personal identification device of the patient comprising a memory wherein at least an identification code of the patient is stored and means for reading and writing said memory, as well as means communicating with further remote operating units upon transmission and/or receiving.

Advantageously in addition to the patient identification code, the memory contains the type of diagnostic and/or therapeutic operation to be carried out by the patient.

In addition, the memory contains data about previous diagnostic tests and/or about therapeutic treatments of the patient, as well as particular pathologic conditions of the patient, which data are read by one or more operating units within the network, the correspondence of the used drug, of operating settings and of dispensing protocols with the predetermined ones for diagnostic and/or therapeutic operations to be carried out being checked, as well as the right choice of the type of diagnostic and/or therapeutic operation to be carried out on the patient being checked with the identification code stored in the identification device.

By means of this characteristic the safety of the patient is more increased and costs are optimized, since possibly harmful operations for the patient and the repetition of diagnostic tests or therapeutic operations that are wrong or carried out in a wrong way are avoided.

Characteristics of the invention will be more clear from the following description of some embodiments shown in annexed drawings wherein:
Fig.1 is a flow diagram of a first embodiment of the device according to the present invention.
Fig.2 is again a flow diagram of a further variant embodiment of the device according to the present invention.
Fig.3 is a flow diagram of a device example according to the present invention and wherein said device has a structure of the communication network type or the like.
Fig.4 is a diagram of communication connections among different units composing the diagnostic/therapeutic device with a network-like structure.

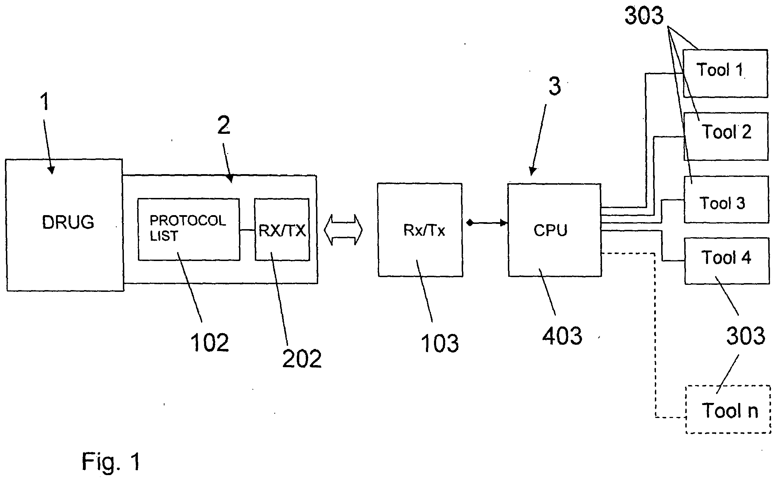

With reference to figure 1, there is shown a flow diagram of a drug in a container to which a so called RFID tag (radio Frequency Identification tag) is associated, i.e. a radio frequency identification chip in the form of a tag. Said RFID tag chip comprises an Rx/Tx receiving/transmitting unit denoted by 202 and a memory 102 wherein different types of data can be stored, such as information about the kind of drug as regards biochemical point of view, origin information for the certificate of quality and originality of the drug and other similar data among the others, as well as data relevant to one or more protocols for using the drug that can be displayed on a suitable reader and/or can be automatically loaded by a suitable reader as configuration files or as driver files of an intelligent or automatic or semi-automatic device dispensing said drug.

In figure 1 said condition is generally shown by means of a generic operating unit denoted by 3 comprising a transmitting/receiving unit 103 by which it communicates with the RFID unit 2 and it is managed by a central unit 403 intended to read and process data retrieved from the memory 102 of the RFID unit 2. The central unit 403 can communicate with or can control one or more operating tools 303. It is possible to provide any type of operating tools 303 and operating tools can be intended to carry out different tasks.

An operating tool for example can be a simple displaying device allowing the user to read data contained in the memory of the RFID unit 2. A different tool can be means for inputting information that in turn can be transmitted to the RFID unit 2 and can be written in the memory 102 thereof. One or more further tools can be intended to perform one or more operations regarding said drug and they can comprise the fact of drawing the drug from the container and/or dosing and/or dispensing the drug to the patient according to different modalities provided for the dispensing operation and particularly for example by means of a direct injection by an automatic/robotized syringe and/or also by dispensing the predetermined dose of the drug and with predetermined dispensing time in a container to which an invasive dispensing system is connected such as a catheter or the like. Further operating tools that can be provided as an alternative or in combination with preceding tools can be intended for example for carrying out diagnostic and/or therapeutic and/or surgical operations on the patient dispensed with the drug.

Particularly as regards different modalities for dispensing the drug, with respect to dosage and/or dispensing action and as regards the modality for carrying out diagnostic or therapeutic operations on the patient dispensed with the drug, at least a protocol for using and/or dispensing the drug can be stored in the memory 102 of the RFID unit 2. Often drugs can be used and/or dispensed in different ways depending on the type of disease and/or the type of patient and/or the type of anatomical region or tissue that is treated thereby. In this case, the memory of the RFID tag 2 can have various protocols for dispensing and/or using the drug under different conditions.

Above mentioned protocols can be simple alphanumeric indications i.e. texts that are displayed and that are carried out or executed by the user or they can be also files configurating or setting one or more tools that are involved in a predetermined therapeutic and/or diagnostic and/or surgical operation that are automatically read by operating tools 303 intended to carry out said operations and which operating units draw data or parameters setting their operating modality directly from the protocol file and they set theirselves on the basis of said data.

It is also possible for said functionalities i.e. the one limited to merely inform the user and so substantially having an indirect effect on operating tools involved in the diagnostic and/or therapeutic and/or surgical operation and the one limited to automatically set at least a part or all operating tools to be provided in combination therefore the user can also possibly force automatic settings by personalized ad hoc settings.

Under this configuration, the drug-container unit with RFID chip is a kind of operating unit that is added to other operating units 303 under the same hierarchical rank while providing use/dispensing drivers allowing all operating units or tools 303 to be operated and controlled in a way consistent with characteristics of the drug and with modalities for using it, as well as with purposes for using the drug. If the above variant is considered wherein parameters configurating and setting individual tools 303 and information controlling them are read from the list of protocols, so the drug-container unit with RFID chip has to be considered like an hardware having its own drivers in the form of software automatically installing itself.

The embodiment of figure 1 provides a first step wherein the drug 1 and container unit with RFID chip 2 is provided in combination with a single device for the use that can be relatively complex and can be provided with various tools, but it is a single device such as for example an apparatus for diagnostic activities and/or therapeutic activities and/or surgical activities.

In figure 1 there is shown a more specific embodiment composed of a robotized or automatic dispenser for the drug, for example a robotized syringe.

In this case, the drug 1 is provided in a predetermined container to which the RFID chip 2 is associated having a memory 102 for one or more lists of protocols for dispensing/using the drug and a transreceiving unit 202. The robotized syringe has an actuator for drawing predetermined amounts of the drug from the container, which actuator 303 draws a predetermined amount of drug, for example in liquid state from the container by a suction duct. The actuator 303 provides a predetermined drug dose to a dispenser 803 that is another tool of the robotized syringe and having a dispensing/injection catheter or a needle, a cannula for example connected to the actuator 303.

The actuator 303 and the dispenser 803 are controlled by a central control unit 403 communicating with the RFID chip 2 by a transreceiving unit 103. A program controlling the actuator 303 and the dispenser 803 is loaded in the central control unit 403 and the central control unit 403 executes said control program.

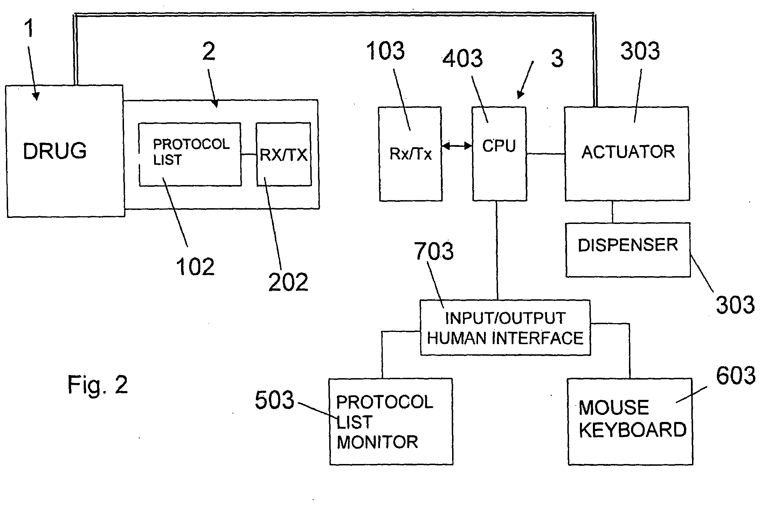

The logic control program provides all control procedures or routines of functions for operating and deactivating the actuator 303 and the dispenser 803, as well as the transreceiving unit 103 and possibly also an input/output unit 703 allowing the operating personnel to communicate with the device by input means such as for example a keybord, a mouse, a joystick, a microphone and a voice recognition unit, a displaying monitor and other similar devices. These can be provided both in a separated way and in any combination and in figure 2 they are denoted by the box 703 and by specific units relevant to the monitor 503 and the keybord and the mouse 603.

Under the control of the logic control program the central control unit 403 provides to execute procedures for recognizing the presence of the drug and for reading information contained in the memory 102 of the RFID chip 2. In this case data that can be retrieved can be data identifying the drug, its expiry date, the composition for example as regards the amount of active principle provided in the formulation, as well as data verifying the orginality of the drug such as data about the origin and other critical identification data. In addition to these data, the central control unit 403 from the memory 102 provides to read the protocol or protocols for using and dispensing the drug.

The latter can be at least partially automatically read as regards parameters controlling the actuator and/or the dispenser that for example are generally used by all or most applications of the drug 1. These data or parameters specifically complete the program controlling tools such as the actuator 303 and the dispenser 803 for the drug being used. Some of these data and particularly data or parameters for using/dispensing the drug that are dependant from the patient condition such as for example the anatomical region to be treated, from the kind of disease and/or the gravity of the damage and from other conditions can be provided for the manual selection by the user by means of the monitor. In this case the control program acts in order that possible choices appear on the monitor and it waits for choices by the operating personnel for example by means of the mouse or the keyboard.

Once said choices have been made the program reads and loads setting and operating parameters and data selected by the user and it controls the actuator 303 and the dispenser 803 such to carry out the action dispensing the drug by doses and modalities provided by protocols stored in the RFID chip 2.

A variant to this operating modality can provide the user to input specific information regarding the patient condition such as the anatomical region to be treated, the kind of disease and/or the gravity of the damage and other conditions before protocols are read and therefore it can provided the protocol reading and the drawing of data and parameters setting and controlling tools 303 and 803 to automatically occur. The high flexibility of the described system allows for example in this case to provide automatic confirmation warnings by the central control unit 403 for example by the monitor 503 and by means of speech synthesis from a loudspeaker. These warnings can sum up selected settings and can inform the operating personnel about the fact that the disease to be treated is within a certain category or that the treatment set in this way requires some attention due to the potential danger or due to other risk factors.

A further improvement of the present invention provides the drug and container unit with RFID chip 2 to be used not only with a single device, but with a plurality of devices.

A typical but not limitative situation is for example with imaging with constrast media. In this case, contrast media have to be injected at predetermined amounts, in predetermined regions, modalities and time. Moreover also the image acquisition has to be carried out according to predetermined modalities both regarding the setting of the imaging machine and as regards time. So for example in the case of ultrasound images it is necessary to set different and predetermined soundproofing powers for each different kind of contrast medium. Moreover the ultrasound machine is set in a different way depending if perfusion measurements or simple diagnostic imaging is desired to be carried out.

In this simple case, it is possible to provide both the robotized dispenser, i.e. the robotized syringe and the diagnostic imaging machine to be provided with a central control unit 403 with a transreceiving unit able to communicate with the transreceiving unit 202 of the RFID chip associated to a predetermined contrast medium. The central control unit of the robotized syringe and also the one of the diagnostic imaging machine execute corresponding control programs that act in a similar way that is they allow protocols for using and dispensing the contrast medium to be read and allow to manually or automatically or partially manually or automatically draw data and parameters setting and operating the robotized syringe for injecting the contrast medium and the diagnostic imaging machine such as described above with reference to preceding examples of figures 1 and 2. Therefore in this case the drug 1 with the container and the RFID chip 2 is an operating unit both of the robotized syringe and of the imaging machine and a kind of network for the communication among said machines is obtained.

The arrangement described above can provide a plurality of machines all communicating with the drug 1 and container operating unit with RFID chip 2. Different modalities can be provided in order to allow the communication among different machines and the drug 1 and container unit with RFID chip. So for example it is possible to provide the device dispensing the drug, such as the robotized syringe or the like, to be a kind of server communicating with the drug 1 and container unit with RFID chip 2. In this case, individual machines for carrying out diagnostic, therapeutic and/or surgical activities related to the dispensing of the drug 1 communicate with the RFID chip by the central control unit 403 of the robotized syringe and at least a part of the control of said machines can be taken by said control unit 403 of the robotized syringe or other machine dispensing the drug.

Also in this case, all setting and operating modality data and parameters of all or of a part of machines can be completely or partially manually defined by the user and/or completely or partially automatically defined like what has been described with reference to preceding examples or by similar modalities.

With reference to the preceding example in combination with the drug robotized dispenser and with one or more machines performing diagnostic, therapeutic and/or surgical activities a patient management unit can be also provided, such as the inner information network of an hospital, a clinic or doctor surgery. The patient management unit can be for example composed of a server managing all personal data and case history of individual patients, as well as a list of important diseases the patient has been subjected to, performed tests results of said tests and therapeutic and/or diagnostic operations undergone by the patient for at least a time period and moreover the pathology currently suffered by him or diagnostic or control tests to which he has to be subjected.

In this case, the drug 1 and container unit with RFID chip 2 communicates also with said patient management server that obviously is provided with its own transmitting and receiving unit. Like what has been described above in this case, the patient management server can be an operating unit that like other ones can communicate with the RFID chip 2 associated to the drug 1. As an alternative, the patient management server can also execute server tasks for communicating and controlling other robotized operating units or diagnostic and/or therapeutic and/or surgical machines provided for carrying out actvities on the patient.

In both cases it is important to notice how said server can automatically and remotely set choices among different protocols for using and dispensing the drug that in the example of figure 1 and 2 was performed at least partially by the user. Within the server there are stored for each patient information regarding the disease to be treated or diagnostic tests to be carried out and details about patient conditions.

The network of operating units performing different tasks in the medical field both regarding the patient treatment and the management of information like all information networks can be easily enlarged by any kind of units. However considering the fact that as the network and possibilities increase, information to be stored in the protocol or protocols increase it is advantageous to provide a further server acting in the network with other operating units and it is a server managing protocols for dispensing and using the drug. In this case once the drug has been identified and once the kind of patient and the kind of diagnostic, therapeutic and/or surgical activities to be carried out on or with it have been indicated, individual operating units, such as the robotized dispenser, the machine or machines for performing diagnostic, and/or therapeutic and/or surgical activites and/or also the patient management server can be connected to the server managing protocols for using and dispensing the drug to download the protocol corresponding to setting and operating modalities and that are specific for the case being carried out.

By means of this it is also possible to set diagnostic, therapeutic and/or surgical activities related to the dispensing action of the drug in a very precise way for each contingent case.

As an alternative to the fact that all machines communicate with the server managing protocols for dispensing and using the drug it is also possible to provide a control server that provides to communicate with the server managing protocols for dispensing and using the drug and so to send data taken therefrom to various operating units.

The presence of a server managing protocols for dispensing and using the drug allows to have use protocols that are constantly updated, simply by providing operating units and particularly patient management servers to send to the server managing protocol for dispensing and using the drug clinical data obtained by said drug when performing diagnostic and/or therapeutic and/or surgical activities, as well as possible side effects and other phenomena. This allows not to lose important medical information allowing both to increase the drug efficacy and possibilities for using it, and to reduce risks when it is used in particular cases and wherein particular conditions or side effects occur.

In order to made the network executing and controlling diagnostic and/or therpaeutic and/or surgical activites it is possible to provide different kind of networks with any kind of hierarchical structure.

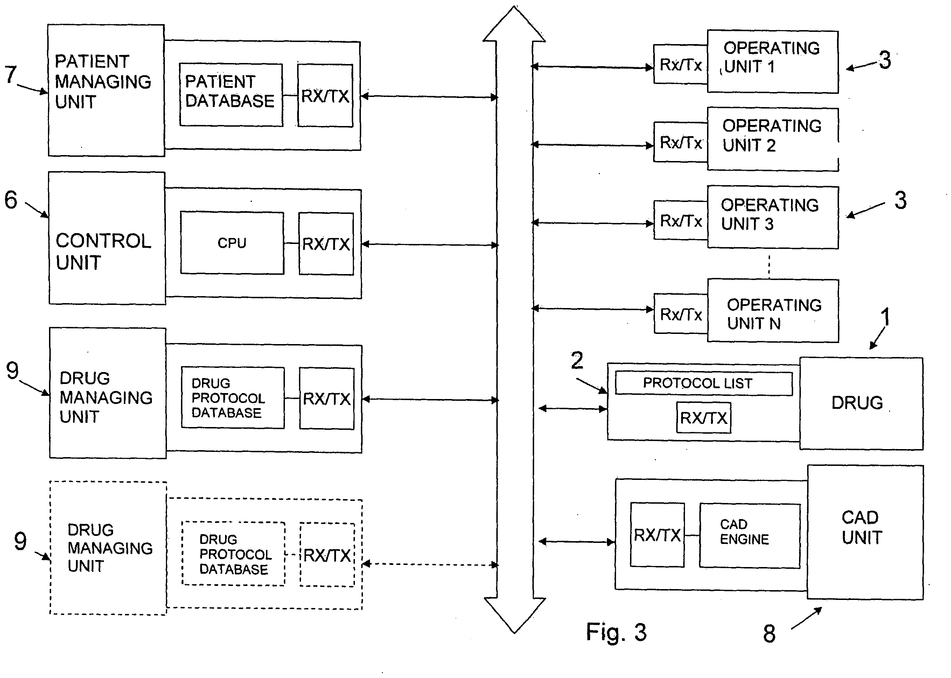

A not limitative example is shown in figure 3. In this figure the communication network that can be a network of the wi-fi type or the like connects according to access criteria that can be defined as one desires and that are determined by opportunity choices and also logic choices, a patient management unit 7 comprising, as already said, all important personal and clinical data of patients, as well as the diagnostic and/or therapeutic and/or surgical activity to which he has to be currently subjected and comprising also a memory area for results of said activity or activities in combination with protocols for dispensing and using the drug used when performing said activities. A control unit 6 is a server reading or retreiving protocols for dispensing and using the drug on the basis of information relevant to clinical conditions of the patient and of diagnostic and/or therapeutic and/or surgical activities to which he has to be subjected under the drug. To this aim the control server 6 is connected to a drug management server comprising a database of protocols for dispensing and using the drug under different clinical conditions of the patient and for carrying out specific diagnostic and/or therapeutic and/or surgical activities. Said server can also comprise a section collecting clinical data obtained during activites related to the dispensing action and use of the drug in order to improve and integrate the database of protocols for dispensing and using the drug on the basis of new use experiences and of results of these use experiences.

This feed-back action to the database managing protocols of the drug or drugs is very important, since by it the drug changes from a simple active principle to an element of a set of use information in various fields and under various conditions, being possible to have access also to use particularities under particular and rare conditions and above all making such knowledge a dynamic one. Particularly the system can provide the protocol, both the one comprised in the memory of the RFID chip or in the remote database and also the protocol that is locally decided by the user and manually set to be transmitted or being possible to be transmitted to the remote unit managing the database of protocols together with clinical and personal information relevant to the disease, the kind of performed test, characteristics of the patient and his identification code and of the doctor or radiologist in charge of the test and such information are put in relation with the specific drug, particularly with the specific contrast medium that has been used.

When there are provided various different kinds of drugs it is possible for the databases of use and dispensing protocols to be managed by different organizations, such as for example pharmaceutical companies producing drugs therefore the network can provided the connection and therefore the access to two or more different servers managing databases of protocols for dispensing and using said different drugs such as schematically indicated by the drug managing unit shown by a discontinuous line and indicated by 9 in figure 3.

Obviously as already described above both in an active and passive way to the network various operating units 3 are connected that are machines intended to perform one or more diagnostic, therapeutic and surgical activities comprising also robotized dispensers or syringes.

Obviously the drug in the form of container and drug 1 unit with RFID chip 2 is also connected to the network.

Still according to a further characteristic of the present invention, a further operating unit can be composed of a CAD unit i.e. for performing so called computer aided diagnostics services and that in figure 3 is indicated by 8.

In this case, also the CAD unit can be set partly by optimized setting data for the drug that is used and for the type of tests that are carried out said data being contained in the protocol or protocols for dispensing and using the drug. Modalities reading and/or transmitting and/or drawing said data performing CAD activities can be similar to the ones already described above for other operating units.

Moreover the CAD unit can be also provided as an additional service to the drug by the company producing or selling the drug, so also in this case, it is possible to improve the efficacy and the reliability of results provided by different systems by means of the constant collection of clinical data and therefore direct clinical experience ones.

The scheme of figure 3 shows again how the container and drug 1 unit with RFID chip 2 changes the conventional drug idea and makes the drug as an element of a set of hardware/software units dedicated to perform biological or medical activities.

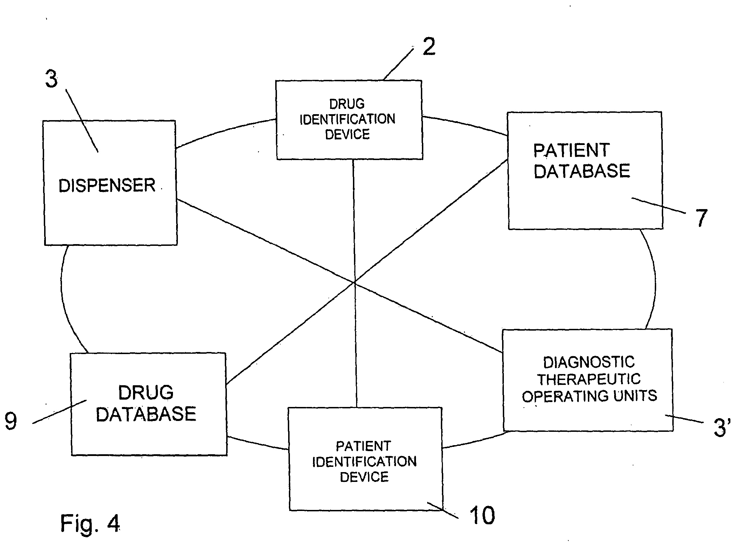

Finally figure 4 shows how the flexibility of the drug and container unit with RFID chip 2 allows to increase not only the accuracy of information about using drugs but also how it is possible to increase the safety of operations providing the use of a drug.

Particularly by providing a suitable RFID chip also on the patient, which chip provides a memory wherein there is stored not only the identification code of the patient, but the kind of operation or operations to which he has to be subjected it is possible to guarantee a complete automatism in selecting the drug, in dispensing it and in using it with reference to the setting of various operating units performing diagnostic and/or therapeutic and/or surgical activites and also with reference to operating units evaluating results of activities such as CAD units. Moreover at the same time the greatest safety against errors in dispensing or using the drug is reached at any extents since by means of the direct identification of the patient it is possible to carry out a cross-verification about the identity of the patient and tests to be carried out.

As regards the practical application of what mentioned above it will be described in the following a practical example of a robotized dispensing system in combination with a ward or hospital information system. In the described example communication means based on a communication protocol called DICOM are used. This protocol is known per se and is widely used in the medical field. More information are contained in PACS and Imaging Informatics: Basic Principles and Applications. H. K. Huang D. Sc. FRCR (Hon.), 1998.

The example particularly refers to a contrast medium to be administered before or during a diagnostic imaging procedure by a robotized injector of the type according to figure 2 in communication for example by WIFI, Bluetooth or another network also by cable with a central information system of the hospital and/or with a local information system of a ward or radiology/imaging department. The communication protocol is the one called DICOM with the aim of downloading a work protocol with data relevant to the patient (i.e. name, weight, allergies, creatinine and other data), data relevant to the procedure to be performed allowing to select among various stored protocols the one that best fits clinical requirements, data relevant to the doctor and/or radiologist in order to fit the use and/or dispensing protocol to his preferences and/or as stored in the memory of the dispenser or injector. Some data such as for example the kind of needle to be used can be loaded before the test in order to further customize protocols.

Once the test of a first patient is completed data of the following patient are loaded and the system automatically get set. Advantageously the system will allow technicians and/or radiologists and/or doctors to have the possibility to change injection and/or performing protocols of the test in order to carry out a fine adjustment of operating modalities and settings of test functionalities and parameters.

Moreover data are sent also to the patient EMR archive such as for example the contrast medium used, the amount of contrast medium and of saline solution, the amount of contrast medium injected per unit of time, the delay of the injection beginning with respect to the test beginning or viceversa and phasing of different operating steps with steps or duration of the injection procedure. During the injection, conditions of the injector can be seen in each moment.

According to a further characteristic, in order to take information about the patient and about the test it is necessary the fact of guaranteeing that the injector CT can take the operating protocol according to the DICOM standard(DICOM Modality Worklist), from the hospital central information system or from the local one and that it can read and manage information contained in said operating protocol.

In order to allow that, the injector CT will have to comprise in the memory an operating protocol software according to DICOM modality (using DICOM Basic Modality Worlist SOP Class as Service Class User-SCU) which will periodically execute (automatically or by the user control) a query (launching the FIND message) in order to obtain a list of procedures provided by the Modality Worklist of the hospital centralized information system or by the ward/department local information system (DICOM Modality Worklist SCP).

The received information is stored in a local database to which the technical personnel can have access in order to identify the subsequent patient and/or procedures to be carried out.

Executed procedures will be removed from the database in the memory of the injector CT when the information system will indicate that no other following tests or no other procedure is provided. The software of the injector CT can comprise protocols for injecting contrast media correlated to the kind of test and/or to an individual or a group of individuals acting like operating personnel. Said software can also compute the volume of contrast media based on the weight, sex and/or other information about the patient and suggest them.

An alternative to the previous method provides the injector to be connected to the network in order to exchange data with a central database. As input said data comprise information relevant to the imaging and/or contrast administration protocol that has been used for the specific test and/or for specific clinical-diagnostic activities or needs and/or for the health history of the patient and for characteristics and/or condition thereof. As output, i.e. from the injector to the central database, they comprise each single used operating protocol and helping at creating a database of protocols WW.

In order to transfer back information relevant to the performing of the actual injection, such as the type and amount of contrast medium, injected amount per unit of time and other data, the injector CT uses a different software application. For this software application there are two alternativesA first alternative provides to use a so called DICOM modality Performed Procedure Step (MPPS) SOP class as Service Class User (SCU). Said MPPS procedure allows to easily transfer procedural data to SCP (Service Class Performee). As soon as the injection has been made, the PPS (Performed Procedure Step) sends a N-CREATE message to the hospital central information system and/or to the ward local information system without any interventions by personnel.

The second DICOM based alternative modality provides to use a different DICOM object and i.e. the so called Structured Report (SR) that is sent to the hospital centralized information system and/or to the local ward information system (which act as DICOM servers in above examples) by means of the DICOM STORE command (SCU class). The advantage of this Structured Report with reference to MPPS is the possibility of storing more complex and structured information such as for example:
" duration of injection (h:m:s); first injected volume, first injected amount per unit of time and/or injection delay; second injected volume; second injected amount per unit of time; used contrast medium."

Up to now IHE have considered the addition of injection data in the MPPS worklist only for the nuclear medicine.

In order to make the injector CT suited to act with a plurality of different CT devices and different hospital centralized information systems and/or different ward local information systems it is advantageous for the DICOM Modality Worlist and the performed Procedure Step and the Structured report to be certified in accordance with DICOM 3.0 standard and to be implemented in accordance with the IHE Technical Framework.

From the above it is clear that with reference to described examples the robotized injector in its memories has to be loaded and it must have the possibility to execute following software applications:
Worklist SCU with protocol database
SR SCU inclusive of the creation and storage of SR object (Structured Report) or MPPS SCU.

In the case if the hospital central information system and/or the ward/department local information system is of the DICOM type or better it integrates IHE, this sytem is able to receive the DICOM SR object or MPPS message.

In the case if the hospital central information system and/or the ward local information system is not of the DICOM type, so there is the need to install a separated unit, such as a computer a PC or the like with DICOM ability and receiving information from the injector and transforming them in a format that can be read by the hospital central information system and/or by the ward local information system.

For the above it is important to have the possibility of having an integrated IHE profile for both applications. Therefore, advantageously, the DICOM Modality Worklist software is integrated with a IHE Scheduled Workflow Integration profile, while as regards the SR (Structured Report), it can be associated to a profile of the type IHE Simple Image and Numeric Report like data relevant to the diameter of vessels shown in images in order to guarantee the connection with IHE integrated hospital central information system and/or ward local information system and/or PACS.

## Claims

1. Therapeutic and/or diagnostic network comprising:
at least a means containing at least a drug or a contrast medium (1) a drug or contrast medium identification device (2) comprising at least a memory (102), means for reading and writing said memory and transmitting and receiving means (202);
said identification device (2) being univocally associated to the means containing the drug or contrast medium (1); information about said drug or contrast medium being stored in said memory (102);
at least an operating unit (3) for using said drug or contrast medium;
said operating unit (3) comprising receiving and transmitting means (103) intended for communicating with receiving and transmitting means (202) of the identification device (2) for retrieving information within the memory (102) of said identification device (2);
wherein the memory (102) of the identification device (2) has at least a protocol for administrating and dispensing the drug or contrast medium that is automatically retrieved by said operating unit (3) using the drug or contrast medium, which operating unit comprises one or more tools (303) intended to carry out predetermined tasks on, with or in the presence of said drug or contrast medium and means (403) controlling said tools using the administration and dispensing protocol as a driver controlling at least some of said tools (303),
the drug or contrast medium with the identification device (2) is, as an operating unit of the network, in communication, in a direct or indirect way, with two or more further operating units (3, 3', 6, 7, 9) having different tasks and being intended to be used for carrying out a diagnostic and/or therapeutic operation,
the network comprising:
a central unit (7) managing information about each patient comprising means (107) for the communication with the identification device (2) and/or with one or more operating units (3) in a direct way or by means of other local communication units;
an operating unit (9) managing a drug or contrast medium database comprising a memory wherein the drug or contrast medium database is stored and corresponding administration, dispensing and use protocols, a central unit (109) having access to data of said database and means (209) for the communication of said operating unit (9) managing the drug database with one or more of the further operating units (3, 3') and with the identification device (2);
a logic program controlling the functional interaction between the drug or contrast medium, the device identifying it, dispensing means and/or one or more further operating units (3, 3'), and which logic program can be executed by the operating units (3, 3', 6, 7, 9) or by a central control unit,
wherein
the identification device (2) is configured to furnish the drug or contrast medium identification code;
the central unit (7) managing the patient database is configured to furnish the kind of operation to be carried out and the characteristics of the patient, as well as possibly the kind of operating units (3, 3') necessary for performing the operation to be carried out; the operating unit managing the drug database (9) is configured to receive said information and furnish in turn to the operating unit (3) dispensing the drug or contrast medium and to different operating units (3'), protocols for administrating, dispensing and using the drug or contrast medium and protocols for setting and controlling different operating units (3') for performing diagnostic and/or therapeutic operations in combination with said drug or contrast medium respectively in a direct way or by a central control unit (6),
wherein
the operating unit (9) managing the drug or contrast medium database is constituted by a server wherein a drug or contrast medium database is stored or by a plurality of servers, which server or servers are remote computers placed by various drug manufacturing companies and allow the access to the database as an additional service for the drug or contrast medium;
said server comprising a section collecting clinical data obtained during activities related to the dispensing action and use of the drug or contrast medium in order to improve and integrate the database of protocols for dispensing and using the drug or contrast medium on the basis of new use experiences and of results of these use experiences,
**characterized in that**
the network comprises a CAD processing unit (8) for processing and evaluating diagnostic data in an automatized way;
wherein the CAD processing unit (8) is a local server wherein a dedicated CAD processing program is loaded, such program being contained in the memory (102) of the identification device (2).

2. Network according to claim 1, **characterized in that** the operating units (3, 3') are hardware/software systems comprising a control unit (403) and one or more peripherals (303) intended for carrying out predetermined tasks and which peripherals (303) are controlled by the control unit (403) by a general control software, while specifications for operating said peripherals (303) are contained in administration and dispensing protocols that therefore are equal to or can be considered equal to drivers of computer peripherals or the like

3. Network according to claim 1 or 2, **characterized in that** the operating units are of the automatic or semi-automatic type and are composed of a computer comprising a central processing and control unit and a plurality of peripheral units, a general program for controlling individual peripherals being stored in the central processing and control unit, while the identification device (2) is a further peripheral that automatically provides drivers for using it comprising information for controlling further peripherals according to operating protocols for the drug or contrast medium.

4. Network according to one or more of the preceding claims, **characterized in that** in the memory (102) of the identification device (2) associated to the drug or contrast medium can store two or more different administration and dispensing protocols or two or more different use protocols for the drug or contrast medium for two or more different use conditions, further comprising a peripheral (4) for displaying and selecting the protocol to be used, which peripheral is configured to carry out such selection manually or by auomatic means to cause the selected protocol to be loaded in means (403) controlling the operating unit (3).

5. Network according to claim 4, **characterized in that** means (403) controlling the operating unit (3, 3') is configured to read the memory of identification devices (2) associated to drugs or contrast media and display use protocols, while the operating unit (3, 3') comprises a displaying screen (503) and means (603) for selecting and inputting commands.

6. Network according to any preceding claim, **characterized in that** it is composed of an identification device unit (1,2), a dispensing unit (3) and at least a further local operating unit (3') or also two or more different local operating units (3') that are intended to carry out different operations on, with or in the presence of the drug or contrast medium.

7. Network according to any preceding claim, **characterized in that** one or more of said local operating units (3), in a direct way and/or by a further central control unit intended to act in combination with the first one (3), can read protocols for administrating, dispensing and using the drug or contrast medium, which in this case contain specific information for controlling all or at least a part of the local operating units (3, 3') intended to act in combination one with the other.

8. Network according to one or more of the preceding claims, **characterized in that** at least one of the operating units (3, 3') is composed of an automatic or semi-automatic dispenser, and/or a diagnostic apparatus and/or an apparatus for acquiring diagnostic images and/or an apparatus for carrying out therapeutic operations.

9. Network according to one or more of the preceding claims, **characterized in that** a first operating unit is intended for delivering contrast media in an automatized way, a second operating unit (3') being composed of at least an image acquisition machine, which acquisition is syncronized and provides predetermined acquisition steps as well as predetermined acquisition setting parameters and that are specifically set for the kind of test and/or anatomical region and organ, data for administrating, dispensing the drug and for performing the diagnostic test being taken from administration, dispensing and performing protocols for the diagnostic test furnished by the drug identification device (2), said operating unit having means for displaying said protocols and means for selecting one of said protocols by the user and therefore means transmitting and loading individual protocols in the dispensing unit and in the unit performing the diagnostic test as specialized control drivers.

10. Network according to one or more of the preceding claims, **characterized in** further comprising means for pre-setting the modality for using the drug or contrast medium such as the anatomical region or organ wherein the drug or contrast medium has to be dispensed, and the type of diagnostic test, said information being able to be directly set in the control unit (403) of one of the local operating units and/or of all local operating units (3, 3'), said setting on how to use the drug or contrast medium being used as an automatic control selecting the specific protocol for administrating and dispensing and using the drug or contrast medium for different local operating units provided when performing the pre-set diagnostic test.

11. Network according to one or more of the preceding claims, **characterized in that** it comprises means for detecting settings operating different local operating units (3, 3') and comparing means which is configured to compare said settings with specific settings defined in protocols for using the drug or contrast medium stored in the identification device (2), which comparing means is configured to generate an alarm signal or automatically change settings operating the operating unit or units (3, 3') when said comparison detects a difference between settings defined in protocols for administrating, dispensing and using the drug and operating settings set in the local operating unit or units (3, 3').

12. Network according to one or more of the preceding claims, **characterized in** comprising a central control unit (6) composing the centralized receiving/transmitting station and communicating both with the identification device and with one or more or all local operating units (3, 3'), and it is configured to exchange parameters for administrating, dispensing or using the drug of the protocols stored in the identification device (2) with different local operating units (3, 3'), and it possibly comprises said comparing means, said warning means and said means automatically changing settings of operating units (3, 3').

13. Network according to one or more of the preceding claims, **characterized in that** it has a structure of the client-server type.

14. Network according to one or more of the preceding claims, **characterized in that** it has a structure of the peer-to-peer type or the like.

15. Network according to any preceding claim, **characterized in that** said central patient management unit (7) comprises personal databank of patients wherein diagnostic and therapeutic information and possible controindications conditions with reference to specific drugs or contrast medium are associated to each patient, said information being used as a means for automatically selecting the diagnostic test and/or the therapeutic action to be carried out and so as a means also for automatically selecting protocols for administrating and dispensing the drug or contrast medium and setting protocols operating local operating units (3, 3') necessary to carry out diagnostic or therapeutic operations.

16. Network according to one or more of the preceding claims, **characterized in that** the local operating unit or units (3, 3') dispensing the drug or contrast medium and performing operations transmit dispensing and operating setting protocols used in performed operations to the central unit (7) managing the patient database which protocols are stored in the patient database together with performed operations.

17. Network according to any preceding claim, **characterized in that** two or more different servers managing the drug or contrast medium database for two or more different drugs or contrast media are connected or can be connected to the network.

18. Network according to one or more of the preceding claims, **characterized in that** the patient managing unit (7) and/or local operating units (3') and/or the central control unit (6) are configured to automatically transmit clinical data for using, dispensing and/or administrating the drug or contrast medium different to the ones indicated in known protocols to the unit (9) managing the drug or contrast medium database.

19. Network according to one or more of the preceding claims, **characterized in that** it comprises a further operating unit constituted by a personal identification device (10) of the patient comprising a memory wherein at least a patient identification code is stored and means for reading and writing said memory, as well as means for communicating with further remote operating units upon transmission and/or receiving.

20. Network according to claim 21, **characterized in that** in addition to the patient identification code, the memory contains the type of diagnostic and/or therapeutic operation the patient has to carry out.

21. Network according to claim 21 or 22, **characterized in that** the memory contains data about previous diagnostic tests and/or about therapeutic treatments of the patient, as well as particular pathologic conditions of the patient, which data are read by one or more operating units (3, 3', 6, 7, 8, 9) within the network, the network being configured to check the correspondence of the used drug or contrast medium, of operating settings and of dispensing protocols with the predetermined ones for diagnostic and/or therapeutic operations to be carried out, as well as to check the right choice of the type of diagnostic and/or therapeutic operation to be carried out on the patient being checked with the identification code stored in the identification device (10).

22. Network according to one or more of the preceding claims, **characterized in that** it is configured to transmit, to the remote server managing the drug protocol database for integrating said database, information about the used protocol both manually and automatially set and/or personal information of the patient and/or information about the medical history and the disease of the patient and/or information about the used drug and dispensing modalities and/or information about the diagnostic and/or therapeutic and/or surgical activity and results of said activity or activities and/or information about the modality processing detected diagnostic data and/or information about personnel carrying out one or more of said units.

## Patentansprüche

1. Therapeutisches und/oder diagnostisches Netzwerk umfassend:
mindestens ein Mittel enthaltend mindestens ein Arzneimittel oder ein Kontrastmittel (1),
eine Arzneimittel- oder Kontrastmittelerkennungsvorrichtung (2) mit mindestens einem Speicher (102), Mittel zum Lesen und Schreiben des Speichers sowie Sende- und Empfangsmittel (202);
die Erkennungsvorrichtung (2) ist eindeutig dem das Arzneimittel oder Kontrastmittel (1) enthaltenden Mittel zugeordnet;
in dem Speicher (102) werden Informationen über das Arzneimittel oder Kontrastmittel gespeichert;
mindestens eine Bedieneinheit (3) zur Verwendung des Arzneimittels oder Kontrastmittels;
die Bedieneinheit (3) weist Empfangs- und Sendemittel (103) zur Kommunikation mit Empfangs- und Sendemitteln (202) der Erkennungsvorrichtung (2) aufweist, um im Speicher (102) der Erkennungsvorrichtung (2) gespeicherte Informationen abzurufen;
wobei der Speicher (102) der Erkennungsvorrichtung (2) zumindest ein Protokoll zur Verwaltung und Abgabe des Arzneimittels oder Kontrastmittels, das von der Bedieneinheit (3) automatisch abgerufen wird, die das Arzneimittel oder Kontrastmittel verwendet, welche Bedieneinheit ein oder mehrere Werkzeuge (303) zur Durchführung vorbestimmter Aufgaben umfasst, mit oder in Gegenwart von dem Arzneimittel oder Kontrastmittel und Mittel (403) zur Steuerung der Werkzeuge unter Verwendung des Verwaltungs- und Abgabeprotokolls als Treiber, der zumindest einige der Werkzeuge (303) steuert,
das Arzneimittel oder Kontrastmittel mit der Erkennungsvorrichtung (2) in direkter oder indirekter Kommunikation als Bedieneinheit des Netzwerks mit zwei oder mehr weiteren Bedieneinheiten (3, 3', 6, 7, 9) steht, die unterschiedliche Aufgaben haben und dazu bestimmt sind, diagnostische und/oder therapeutische Funktionen durchzuführen,
wobei das Netzwerk umfasst:
eine Zentraleinheit (7) zur Verwaltung von Informationen über jeden Patienten mit Mitteln (107) zur Kommunikation mit der Erkennungsvorrichtung (2) und/oder mit einer oder mehreren Bedieneinheiten (3) direkt oder mittels anderer lokaler Kommunikationseinheiten;
eine Bedieneinheit (9) zur Verwaltung einer Arzneimittel- oder Kontrastmitteldatenbank mit einem Speicher, in dem die Arzneimittel- oder Kontrastmitteldatenbank gespeichert ist, und entsprechende Verwaltungs-, Abgabe- und Nutzungsprotokolle,
eine Zentraleinheit (109), die auf Daten der Datenbank zugreifen kann und Mittel (209) zur Kommunikation der zum Verwalten der Medikamentendatenbank vorgesehenen Bedieneinheit (9) mit einer oder mehreren der weiteren Bedieneinheiten (3, 3') und mit der Erkennungsvorrichtung (2);
ein Logikprogramm, das die funktionelle Wechselwirkung zwischen dem Arzneimittel oder Kontrastmittel, der diese erkennenden Erkennungsvorrichtung, Ausgabemitteln und/oder einer oder mehreren weiteren Bedieneinheiten (3, 3')steuert, und das Logikprogramm von den Bedieneinheiten (3, 3', 6, 7, 9) oder von einer zentralen Steuereinheit ausführbar ist,
wobei die Erkennungsvorrichtung (2) zur Bereitstellung des Arzneimittel- oder Kontrastmittel-Identifikationscodes eingerichtet ist;
die die Patientendatenbank verwaltende Zentraleinheit (7) zur Bereitstellung der Typologie der durchzuführenden Aufgabe und der Eigenschaften des Patienten, sowie ggf der Typologie der Bedieneinheiten (3, 3'), die zur Durchführung des auszuführenden Aufgabe notwendig ist;
die die Arzneimitteldatenbank (9) verwaltende Bedieneinheit ist dazu eingerichtet, diese Informationen zu empfangen und wiederum an der Bedieneinheit (3) bereitzustellen, die zur Abgabe des Arzneimittel- oder Kontrastmittels vorgesehen ist, und an unterschiedliche Bedieneinheiten (3'), Protokollen zur Verwaltung, Abgabe und Verwendung des Arzneimittels oder Kontrastmittels und Protokollen zur Einstellung und Steuerung verschiedener Bedieneinheiten (3') zur Durchführung von diagnostischen und/oder therapeutischen Funktionen in Kombination mit dem Arzneimittel oder Kontrastmittel jeweils direkt oder durch eine zentrale Steuereinheit (6),
wobei die die Arzneimittel- oder Kontrastmitteldatenbank verwaltende Bedieneinheit (9) durch einen Server gebildet ist, in dem eine Arzneimittel- oder Kontrastmitteldatenbank gespeichert ist oder durch mehrere Server, wobei der Server oder die Server entfernte Computer sind, die von verschiedenen Arzneimittelherstellungsfirmen eingerichtet sind und den Zugriff auf die Datenbank als einen zusätzlichen Dienst für das Arzneimittel oder Kontrastmittel ermöglichen;
der Server weist eine Sektion auf, in der klinische Daten gesammelt sind, die bei Aktivitäten erhalten werden, die sich auf die Abgabefunktion und die Verwendung des Arzneimittels oder Kontrastmittels beziehen, um die Datenbank von Protokollen zur Abgabe und Verwendung des Arzneimittel- oder Kontrastmittels auf der Basis neuer Nutzungserfahrungen und Ergebnissen dieser Nutzungserfahrungen zu verbessern und integrieren,
**dadurch gekennzeichnet, dass**
das Netzwerk eine CAD-Verarbeitungseinheit (8) zur automatisierten Verarbeitung und Auswertung von Diagnosedaten umfasst;
wobei die CAD-Verarbeitungseinheit (8) ein lokaler Server ist, auf den ein eigenes CAD-Verarbeitungsprogramm geladen wird, wobei das Programm in dem Speicher (102) der Erkennungsvorrichtung (2) enthalten ist.

2. Netzwerk nach Anspruch 1, **dadurch gekennzeichnet, dass** die Bedieneinheiten (3, 3') Hardware/Software-Systeme sind, mit einer Steuereinheit (403) und einem oder mehreren Peripheriegeräten (303), die zur Durchführung vorbestimmter Aufgaben bestimmt sind und welche Peripheriegeräte (303) von der Steuereinheit gesteuert werden (403) durch eine Hauptsteuersoftware, während Spezifikationen zum Betreiben der Peripheriegeräte (303) in Verwaltungs- und Abgabeprotokollen enthalten sind, die somit gleich sind oder als gleich betrachtet werden zu Treibern von Computerperipheriegeräten oder dergleichen.

3. Netzwerk nach Anspruch 1 oder 2, **dadurch gekennzeichnet, dass** die Bedieneinheiten vom automatischen oder halbautomatischen Typ sind und bestehen aus einem Computer, der eine zentrale Verarbeitungs- und Steuereinheit und mehrere Peripherieeinheiten umfasst, wobei in der zentralen Verarbeitungs- und Steuereinheit ein Hauptprogramm zur Steuerung einzelner Peripheriegeräte gespeichert ist, während die Erkennungsvorrichtung (2) ein weiteres Peripheriegerät ist, das automatisch Treiber zu seiner Verwendung vorsieht und Informationen zur Steuerung weiterer Peripheriegeräte nach Betriebsprotokollen für das Arzneimittel oder Kontrastmittel bereitstellt.

4. Netzwerk nach einem oder mehreren der vorhergehenden Ansprüche, **dadurch gekennzeichnet, dass** in dem Speicher (102) der Erkennungsvorrichtung (2), die dem Arzneimittel oder Kontrastmittel zugeordnet ist, zwei oder mehrere unterschiedliche Verwaltungs- und Abgabeprotokolle oder zwei oder mehr unterschiedliche Verwendungsprotokolle für das Arzneimittel oder Kontrastmittel für zwei oder mehr unterschiedliche Verwendungsbedingungen, ferner umfassend ein Peripheriegerät (4) zum Anzeigen und Auswählen des zu verwendenden Protokolls, welches Peripheriegerät dazu eingerichtet ist, eine solche Auswahl manuell oder durch automatische Mittel auszuführen, um zu bewirken, dass das ausgewählte Protokoll in Mittel (403) geladen wird, die die Bedieneinheit (3) steuern.

5. Netzwerk nach Anspruch 4, **dadurch gekennzeichnet, dass** Mittel (403), die die Bedieneinheit (3, 3') steuern, dazu eingerichtet sind, den Speicher von Erkennungsvorrichtungen (2) zu lesen, die dem Arzneimittel oder Kontrastmittel und Anzeigebenutzungsprotokollen zugeordnet sind, während die Bedieneinheit (3, 3') einen Anzeigebildschirm (503) und Mittel (603) zum Auswählen und Eingeben von Befehlen umfasst.

6. Netzwerk nach dem vorhergehenden Anspruch, **dadurch gekennzeichnet, dass** es aus einer Erkennungsvorrichtungseinheit (1,2), einer Abgabeeinheit (3) und mindestens einer weiteren lokalen Bedieneinheit (3') oder auch aus zwei oder mehr unterschiedlichen lokalen Bedieneinheiten (3') besteht, die dazu vorgesehen sind, unterschiedliche Operationen auszuführen, mit oder in Gegenwart von dem Arzneimittel oder Kontrastmittel.

7. Netzwerk nach dem vorhergehenden Anspruch, **dadurch gekennzeichnet, dass** eine oder mehrere der lokalen Bedieneinheiten (3) in direkter Weise und/oder durch eine weitere zentrale Steuereinheit, die dazu bestimmt ist, in Kombination mit der ersten (3) zu wirken, imstande sind, Protokolle zur Verwaltung, Abgabe und Verwendung des Arzneimittels oder Kontrastmittels zu lesen, die in diesem Fall spezifische Informationen zur Steuerung aller oder zumindest eines Teils der lokalen Bedieneinheiten enthalten (3, 3'), die dazu bestimmt ist, in Kombination miteinander zu wirken.

8. Netzwerk nach einem oder mehreren der vorhergehenden Ansprüche, **dadurch gekennzeichnet, dass** zumindest eine der Bedieneinheiten (3, 3') aus einem automatischen oder halbautomatischen Spender und/oder einer Diagnosevorrichtung und/oder einer Vorrichtung zur Erfassung von Diagnosebildern und/oder einer Vorrichtung zur Durchführung therapeutischer Aufgaben besteht.

9. Netzwerk nach einem oder mehreren der vorhergehenden Ansprüche, **dadurch gekennzeichnet, dass** eine erste Bedieneinheit dazu vorgesehen ist, Kontrastmittel automatisiert abzugeben, eine zweite Bedieneinheit (3'), die aus zumindest einer Bilderfassungsmaschine zusammengesetzt ist, wobei die Erfassung synchronisiert ist und vorgegebene Erfassungsschritte sowie vorgegebene Erfassungseinstellparameter bereitstellt, die spezifisch für eine Typologie von Test und/oder anatomischem Bereich und Organ eingestellt sind, Daten zur Verwaltung, Abgabe des Arzneimittels und zur Durchführung des aus den Verwaltungs-, Abgabe- und Durchführungsprotokollen gewonnenen Diagnosetests für den von der Arzneimittelerkennungsvorrichtung (2) gelieferten Diagnosetest, wobei die Bedieneinheit Mittel zum Anzeigen der Protokolle und Mittel zum Auswählen eines der Protokolle durch den Benutzer und damit Mittel zum Senden und Laden einzelner Protokolle in der Abgabeeinheit und in der den Diagnosetest ausführenden Einheit als spezialisierte Steuertreiber aufweist.

10. Netzwerk nach einem oder mehreren der vorhergehenden Ansprüche, **dadurch gekennzeichnet, dass** es weiterhin umfasst: Mittel zur Voreinstellung der Modalität zur Verwendung des Arzneimittels oder Kontrastmittels wie das anatomische Bereich oder Organ, in dem das Arzneimittel oder Kontrastmittel abgegeben werden muss, und der Typologie des Diagnosetests, wobei diese Informationen direkt in der Steuereinheit (403) einer der lokalen Bedieneinheiten und/oder aller lokalen Bedieneinheiten (3, 3') einstellbar sind, wobei die Einstellung, wie das Arzneimittel oder Kontrastmittel zu verwenden ist, als eine automatische Steuerung verwendet wird, die das spezifische Protokoll zum Verwalten und Abgeben und Verwenden des Arzneimittels oder Kontrastmittels für verschiedene lokale Betriebseinheiten, die bei der Durchführung des voreingestellten Diagnosetests bereitgestellt werden, auswählt.

11. Netzwerk nach einem oder mehreren der vorhergehenden Ansprüche, **dadurch gekennzeichnet, dass** es Mittel zum Erfassen von verschiedene lokale Bedieneinheiten (3, 3') betreibenden Einstellungen und Vergleichsmittel, die dazu eingerichtet sind, diese Einstellungen mit bestimmten in Protokollen definierten Einstellungen zu vergleichen, umfasst, um das in der Erkennungsvorrichtung (2) gespeicherte Arzneimittel oder Kontrastmittel zu verwenden, wobei das Vergleichsmittel dazu ausgebildet ist, ein Alarmsignal zu erzeugen oder die die Bedieneinheit bzw. die Bedieneinheiten (3, 3') betreibende Einstellungen automatisch zu ändern, wenn der Vergleich eine Differenz zwischen den in den Protokollen zur Verwaltung, Abgabe und Verwendung des Arzneimittels definierten Einstellungen und den in der/den lokalen Bedieneinheit/Bedieneinheiten (3, 3') eingestellten Betriebseinstellungen erfasst.

12. Netzwerk nach einem oder mehreren der vorhergehenden Ansprüche, **dadurch gekennzeichnet, dass** es umfasst: eine zentrale Steuereinheit (6), die die zentrale Empfangs-/Sendestation bildet und sowohl mit der Erkennungsvorrichtung als auch mit einer oder mehreren oder allen lokalen Bedieneinheiten (3, 3') kommuniziert, und dazu eingerichtet ist, Parameter zur Verwaltung, Abgabe oder Verwendung des Arzneimittels der in der Erkennungsvorrichtung (2) gespeicherten Protokolle mit unterschiedlichen lokalen Bedieneinheiten (3, 3') auszutauschen und ggf. diese Vergleichsmittel umfasst, wobei die Warneinrichtung und die besagten Mittel die Einstellungen der Bedieneinheiten (3, 3') automatisch ändern.

13. Netzwerk nach einem oder mehreren der vorhergehenden Ansprüche, **dadurch gekennzeichnet, dass** es eine Client-Server-Struktur aufweist.

14. Netzwerk nach einem oder mehreren der vorhergehenden Ansprüche, **dadurch gekennzeichnet, dass** es eine Peer-to-Peer-Struktur oder dergleichen aufweist.

15. Netzwerk nach dem vorhergehenden Anspruch, **dadurch gekennzeichnet, dass** die zentrale Patientenverwaltungseinheit (7) eine persönliche Datenbank von Patienten umfasst, wobei jedem Patienten diagnostische und therapeutische Informationen und mögliche Gegenanzeigen-Bedingungen gegenüber bestimmten Arzneimitteln oder Kontrastmitteln zugeordnet sind, wobei diese Informationen als Mittel zur automatischen Auswahl des Diagnosetests und/oder der durchzuführenden therapeutischen Aktion und damit auch als Mittel verwendet werden, zum automatischen Auswählen von Protokollen zum Verwalten und Abgeben des Arzneimittels oder Kontrastmittels und der Einstellprotokolle, die die lokale Bedieneinheiten (3, 3'), die zur Durchführung von diagnostischen oder therapeutischen Operationen notwendig sind, betreiben.

16. Netzwerk nach einem oder mehreren der vorhergehenden Ansprüche, **dadurch gekennzeichnet, dass** die lokale Bedieneinheit bzw. die lokalen Bedieneinheiten (3, 3') zum Abgeben des Arzneimittels oder Kontrastmittels und zum Durchführen von Operationen die Abgabe- und Betriebseinstellungsprotokolle, die in ausgeführten Operationen verwendet werden, an die die Patientendatenbank verwaltende Zentraleinheit (7) übertragen, welche Protokolle zusammen mit den durchgeführten Operationen in der Patientendatenbank gespeichert sind.

17. Netzwerk nach dem vorhergehenden Anspruch, **dadurch gekennzeichnet, dass** zwei oder mehr verschiedene Server, die die Arzneimittel- oder Kontrastmitteldatenbank für zwei oder mehr verschiedene Arzneimittel oder Kontrastmittel verwalten, mit dem Netzwerk verbunden sind oder verbunden sein können.

18. Netzwerk nach einem oder mehreren der vorhergehenden Ansprüche, **dadurch gekennzeichnet, dass** die Patientenverwaltungseinheit (7) und/oder die lokale Bedieneinheiten (3') und/oder die zentrale Steuereinheit (6) sind dazu eingerichtet, klinische Daten zur Verwendung, Abgabe und/oder Verwaltung des Arzneimittels oder Kontrastmittels, welche sich von den in bekannten Protokollen angegebenen Daten unterscheiden, an die Einheit (9) zur Verwaltung der Arzneimittel- oder Kontrastmitteldatenbank automatisch zu übertragen.

19. Netzwerk nach einem oder mehreren der vorhergehenden Ansprüche, **dadurch gekennzeichnet, dass** es eine weitere Bedieneinheit umfasst, die von einer persönlichen Identifikationsvorrichtung (10) des Patienten gebildet ist, umfassend einen Speicher, in dem zumindest ein Patientenidentifikationscode gespeichert ist, und Mittel zum Lesen und Schreiben des Speichers, sowie Mittel zur Kommunikation mit weiteren entfernten Bedieneinheiten beim Senden und/oder Empfangen.

20. Netzwerk nach Anspruch 21, **dadurch gekennzeichnet, dass** der Speicher, neben dem Patientenidentifikationscode, die Typologie des am Patienten auszuführenden Diagnose- und/oder Therapieverfahren enthält.

21. Netzwerk nach Anspruch 21 oder 22, **dadurch gekennzeichnet, dass** der Speicher enthält Daten über vorherige Diagnosetests und/oder über therapeutische Behandlungen des Patienten, sowie bestimmte krankhafte Zustände des Patienten, welche Daten von einer oder mehreren Bedieneinheiten (3, 3', 6, 7, 8, 9) innerhalb des Netzwerks gelesen werden, wobei das Netzwerk eingerichtet ist, die Übereinstimmung des verwendeten Arzneimittels oder Kontrastmittels, der Betriebseinstellungen und der Abgabeprotokollen mit den vorgegebenen Einstellungen zur Durchführung von Diagnose- und/oder Therapieoperationen zu überprüfen sowie die Übereinstimmung der korrekten typologischen Wahl des am Patienten durchzuführenden Diagnose- und/oder Therapieverfahren mit dem in der Identifikationseinrichtung (10) gespeicherten Identifikationscode zu überprüfen.

22. Netzwerk nach einem oder mehreren der vorhergehenden Ansprüche, **dadurch gekennzeichnet, dass** es dazu eingerichtet ist, Informationen über das sowohl manuell als auch automatisch eingestellte verwendete Protokoll und/oder persönliche Informationen des Patienten und/oder Informationen über die medizinische Historie und die Erkrankung des Patienten und/oder Informationen über die verwendeten Arzneimittel-und Abgabemodalitäten und/oder Informationen über die diagnostische und/oder therapeutische und/oder chirurgische Aktivität und Ergebnisse dieser Aktivität oder Aktivitäten und/oder Informationen über die Verarbeitungsmodalität der erfassten Diagnosedaten und/oder Informationen über das eine oder mehrere dieser Einheiten bedienende Personal, an den Fernserver zu übertragen, der die Arzneimittel-Protokolldatenbank zur Integration dieser Datenbank verwaltet.

## Revendications

1. Réseau thérapeutique et/ou diagnostique comprenant :
au moins un moyen contenant au moins un médicament ou un produit de contraste (1) ;
un dispositif d'identification de médicamente ou de produit de contraste (2) comprenant au moins une mémoire (102), des moyens pour la lecture et l'écriture de ladite mémoire et des moyens de transmission et de réception (202);
ledit dispositif d'identification (2) étant associé de façon univoque aux moyens contenant le médicament ou
le produit de contraste (1) ;
les informations relatives audit médicament ou audit produit de contraste étant contenues dans ladite mémoire (102) ;
au moins une unité d'exploitation (3) pour l'utilisation dudit médicament ou dudit produit de contraste ;
ladite unité d'exploitation (3) comprenant des moyens de réception et de transmission (103) destinés à communiquer avec les moyens de réception et de transmission (202) du dispositif d'identification (2) pour récupérer des informations dans la mémoire (102) dudit dispositif d'identification (2) ;
dans lequel la mémoire (102) du dispositif d'identification (2) possède au moins un protocole pour l'administration e la distribution du médicament ou du produit de contraste, qui est automatiquement récupéré par ladite unité d'exploitation (3) employant le médicament ou le produit de contraste, laquelle unité d'exploitation comprend un ou plusieurs instruments (303) destinés à réaliser des tâches prédéterminées, avec ou en présence dudit médicament ou dudit produit de contraste et des moyens (403) de contrôle desdits outils à l'aide du protocole d'administration et de distribution comme pilote de contrôle d'au moins certains desdits outils (303),
le médicament ou le produit de contraste avec le dispositif d'identification (2) est, comme unité d'exploitation du réseau, en communication, de façon directe ou indirecte, avec deux ou plusieurs unités d'exploitation (3, 3', 6, 7, 9) comportant des tâches différentes, et destinées à être utilisées pour réaliser une opération diagnostique et/ou thérapeutique, le réseau comprenant :
une unité centrale (7) pour la gestion des informations sur chaque patient comprenant des moyens (107) pour la communication avec le dispositif d'identification (2) et/ou avec une ou plusieurs unités d'exploitation (3) d'une manière directe ou par le biais d'autres unités de communication locales ;
une unité d'exploitation (9) pour la gestion d'une base de données de médicaments ou de produits de contraste, comprenant une mémoire dans laquelle la base de données de médicaments ou de produits de contraste est stockée, une unité centrale (109) pouvant accéder aux données de ladite base de données et des moyens (209) de communication pour ladite unité d'exploitation (9) gérant la base de données avec une ou plusieurs des autres unités d'exploitation (3, 3') et avec le dispositif d'identification (2) ;
un programme logique pour le contrôle des interactions fonctionnelles parmi le médicament ou le produit de contraste, le dispositif d'identification de celui-ci, les moyens de distribution et/ou une ou plusieurs unités d'exploitation (3, 3'), ledit programme logique pouvant être exécuté par les unités d'exploitation (3, 3', 6, 7, 9) ou par une unité centrale de contrôle, dans lequel le dispositif d'identification (2) est conçu pour fournir le code d'identification du médicament ou du produit de contraste ;
l'unité centrale (7) pour la gestion de la base de données des patients est conçue pour fournir le genre d'opération à effectuer et les caractéristiques du patient, ainsi qu'éventuellement le genre d'unités d'exploitation (3, 3') nécessaires pour l'exécution de l'opération à effectuer ;
l'unité d'exploitation (9) pour la gestion de la base de données des médicaments est conçue pour recevoir lesdites informations et fournir à leur tour à l'unité d'exploitation (3) pour la distribution du médicament ou du produit de contraste et aux différentes unités d'exploitation (3'), des protocoles pour l'administration, la distribution et l'utilisation du médicament ou du produit de contraste et des protocoles pour le réglage et le contrôle de différentes unités d'exploitation (3') pour l'exécution d'opérations diagnostiques et/ou thérapeutiques en combinaison respectivement avec ledit médicament ou produit de contraste de manière directe ou par le biais d'une unité centrale de contrôle (6), dans lequel l'unité d'exploitation (9) pour la gestion de la base de données des médicamentes et des produits de contraste est constituée d'un serveur dans lequel une base de données de médicaments ou de produits de contraste est stockée ou par une pluralité de serveurs, le ou lesdits serveurs, étant des ordinateurs distants disposés par les différents producteurs de médicaments permettent d'accéder à la base de données en tant que service supplémentaire pour le médicament ou le produit de contraste ;
ledit serveur comprenant une section pour la collecte de données cliniques obtenues au cours d'activités liées à l'action de distribution et à l'utilisation du médicament ou du produit de contraste dans le but d'améliorer et d'intégrer la base de données des protocoles pour la distribution et l'utilisation du médicament ou du produit de contraste sur la base de nouvelles expériences d'emploi et des résultats de ces expériences d'emploi,
**caractérisé en ce que**
le réseau comprend une unité de traitement CAD (8) pour le traitement et l'évaluation des données diagnostiques de manière automatisée ;
dans lequel l'unité de traitement CAD (8) est un serveur local dans lequel un programme de traitement CAD dédié est chargé, ledit programme étant contenu dans la mémoire (102) du dispositif d'identification (2).

2. Réseau selon la revendication 1, **caractérisé en ce que** les unités d'exploitation (3, 3') sont des systèmes matériels/logiciels comprenant une unité de contrôle (403) et un ou plusieurs périphériques (303) destinés à effectuer des tâches prédéterminées, lesdits périphériques (303) étant contrôlés par l'unité de contrôle (403) par le biais d'un logiciel de contrôle général, alors que les spécifications pour le fonctionnement desdits périphériques (303) sont contenues dans les protocoles d'administration et de distribution qui, par conséquent, sont comme ou peuvent être considérés comme des pilotes de périphériques d'ordinateur ou similaires.

3. Réseau selon la revendication 1 ou 2, **caractérisé en ce que** les unités d'exploitation sont de type automatique ou semi-automatique, et sont composés d'un ordinateur comprenant une unité centrale de traitement et de contrôle et une pluralité d'unités périphériques, un programme général de contrôle de périphériques individuels étant stocké dans l'unité centrale de traitement et de contrôle, tandis que le dispositif d'identification (2) est un autre périphérique qui fournit automatiquement des pilotes pour son utilisation comprenant des informations pour le contrôle d'autres périphériques suivant des protocoles de fonctionnement pour le médicament ou le produit de contraste.

4. Réseau selon l'une ou plusieurs des revendications précédentes, **caractérisé en ce que** la mémoire (102) du dispositif d'identification (2) associé au médicament ou au produit de contraste peut contenir deux ou plusieurs protocoles d'administration et de distribution différents ou deux ou plusieurs protocoles d'utilisation différents pour le médicament ou le produit de contraste pour deux ou plusieurs conditions d'utilisation différentes, comprenant également un périphérique (4) pour l'affichage et la sélection du protocole à employer, ledit périphérique étant conçu pour effectuer cette sélection de façon manuelle ou par des moyens automatiques, pour faire charger le protocole sélectionné dans des moyens (403) de contrôle de l'unité d'exploitation (3).

5. Réseau selon la revendication 4, **caractérisé en ce que** les moyens (403) de contrôle de l'unité d'exploitation (3, 3') sont conçus pour lire la mémoire des dispositifs d'identification (2) associés aux médicaments ou aux produits de contraste et pour afficher des protocoles d'utilisation, alors que l'unité d'exploitation (3, 3') comprend un écran d'affichage (503) et des moyens (603) pour la sélection et la saisie de commandes.

6. Réseau selon n'importe laquelle des revendications précédentes, **caractérisé en ce qu'**il est composé d'une unité de dispositif d'identification (1, 2), une unité de distribution (3) et au moins une autre unité d'exploitation locale (3') ou également deux ou plusieurs unités d'exploitation locales (3'), destinées à effectuer des opérations différentes sur, avec ou en présence du médicament ou du produit de contraste.

7. Réseau selon n'importe laquelle des revendications précédentes, **caractérisé en ce qu'**une ou plusieurs desdites unités d'exploitation locales (3), de manière directe et/ou par le biais d'une autre unité centrale de contrôle destiné à agir en association avec celle-ci (3), peuvent lire des protocoles d'administration, de distribution et d'utilisation du médicament ou du produit de contraste, qui contiennent, en ce cas, des informations spécifiques pour contrôler la totalité ou au moins une partie des unités d'exploitation locales (3, 3') destinées à agir en association les unes avec les autres.

8. Réseau selon l'une ou plusieurs des revendications précédentes, **caractérisé en ce qu'**au moins une des unités d'exploitation (3, 3') est composée d'un distributeur automatique ou semi-automatique, et/ou d'un appareil diagnostique et/ou d'un appareil pour l'acquisition d'images diagnostiques et/ou d'un appareil pour la mise en oeuvre d'opérations thérapeutiques.

9. Réseau selon l'une ou plusieurs des revendications précédentes, **caractérisé en ce qu'**une première unité d'exploitation est destinée à alimenter le produit de contraste de manière automatisée, une deuxième unité d'exploitation (3') composée d'au moins une machine d'acquisition d'images, l'acquisition étant synchronisée et comportant des étapes d'acquisition ainsi que des paramètres de réglage d'acquisition prédéterminés, définis spécifiquement pour le type de test et/ou de région et d'organe anatomique, des données pour l'administration, et la distribution du médicament et pour effectuer le test diagnostique choisi à partir des protocoles d'administration et de distribution, et l'exécution de protocoles pour le test de diagnostic étant fournies par le dispositif d'identification de médicament (2), ladite unité d'exploitation possédant des moyens pour l'affichage desdits protocoles et des moyens pour la sélection de l'un desdits protocoles par l'utilisateur et donc des moyens de transmission et de chargement des protocoles individuels dans l'unité de distribution et dans l'unité d'exécution du test diagnostique comme des pilotes de contrôle spécialisés.

10. Réseau selon l'une ou plusieurs des revendications précédentes, **caractérisé en ce qu'**il comporte également des moyens pour le pré-réglage du mode d'utilisation du médicament ou du produit de contraste, comme la région anatomique ou l'organe dans lequel le médicament ou le produit de contraste doit être distribué, et le type de test diagnostique, lesdites informations pouvant être définies directement dans l'unité de contrôle (403) de l'une des unités d'exploitation locales et/ou de toutes les unités d'exploitation locales (3, 3'), ledit réglage relatif au mode d'utilisation ou du produit de contraste étant effectué comme un contrôle automatique de sélection du protocole spécifique pour l'administration et la distribution et l'utilisation du médicament ou du produit de contraste pour différentes unités d'exploitation locales mises en oeuvre pour effectuer le test diagnostique prédéfini.

11. Réseau selon l'une ou plusieurs des revendications précédentes, **caractérisé en ce qu'**il comprend des moyens pour la détection des paramètres d'utilisation de différentes unités d'exploitation locales (3, 3') et des moyens de comparaison, conçus pour comparer lesdits paramètres avec des paramètres spécifiques définis dans des protocoles d'utilisation du médicament ou du produit de contraste stockés dans le dispositif d'identification (2), lesdits moyens de comparaison étant conçus pour générer un signal d'alarme ou modifier automatiquement les paramètres de fonctionnement de la ou les unités d'exploitation (3, 3'), lorsque ladite comparaison détecte une différence entre les paramètres définis dans les protocoles pour l'administration, la distribution et l'utilisation du médicament et de paramètres de fonctionnement définis dans l'unité ou les unités d'exploitation locales (3, 3').

12. Réseau selon l'une ou plusieurs des revendications précédentes, **caractérisé en ce qu'**il comprend une unité centrale de contrôle (6) composant la station centralisée de réception/transmission, et communiquant avec le dispositif d'identification et avec l'une ou plusieurs de toutes les unités d'exploitation locales (3, 3'), et conçu pour échanger des paramètres pour l'administration, la distribution ou l'utilisation du médicament des protocoles stockés dans le dispositif d'identification (2) avec des différentes unités d'exploitation locale (3, 3'), et comprenant éventuellement lesdits moyens de comparaison, lesdits moyens d'alerte et lesdits moyens du changement automatique des paramètres des unités d'exploitation (3, 3').

13. Réseau selon l'une ou plusieurs des revendications précédentes, **caractérisé en ce qu'**il comporte une structure de type client-serveur.

14. Réseau selon l'une ou plusieurs des revendications précédentes, **caractérisé en ce qu'**il comporte une structure de type égal-à-égal ou similaires.

15. Réseau selon n'importe laquelle des revendications précédentes, **caractérisé en ce que** la dite unité centrale de gestion de patients (7) comprend une base de données personnelles de patients, dans laquelle à chaque patient sont associés des informations diagnostiques et thérapeutiques et des possibles conditions de contre-indications, lesdites informations étant utilisés comme un moyen de sélection automatique du test diagnostique et/ou de l'action thérapeutique à effectuer et par conséquent également comme moyen de sélection automatique de protocoles pour l'administration et la distribution du médicament ou du produit de contraste et de réglage de protocoles pour le fonctionnement des unités d'exploitation locales (3, 3') nécessaires pour effectuer des opérations diagnostiques ou thérapeutiques.

16. Réseau selon l'une ou plusieurs des revendications précédentes, **caractérisé en ce que** la ou les unités d'exploitation locales (3, 3') pour la distribution du médicament ou du produit de contraste et pour l'exécution des opérations transmettent à l'unité centrale (7) de gestion de la base de données des patients des protocoles de réglage de la distribution et fonctionnement employés dans les opérations effectuées, lesdits protocoles étant stockées dans la base de données des patients avec les opérations effectuées.

17. Réseau selon n'importe laquelle des revendications précédentes, **caractérisé en ce que** les deux ou plusieurs serveurs différents pour la gestion de la base de données des médicaments et des produits de contraste pour deux ou plusieurs différents médicaments ou produits de contraste sont connectés ou peuvent être connectés au réseau.

18. Réseau selon l'une ou plusieurs des revendications précédentes, **caractérisé en ce que** l'unité de gestion des patients (7) et/ou les unités d'exploitation locales (3') et/ou l'unité centrale de contrôle (6) sont conçus pour transmettre automatiquement à l'unité (9) de gestion de la base de données des médicaments ou des produits de contraste des données cliniques pour l'utilisation, la distribution et/ou l'administration du médicament ou du produit de contraste, différentes des données indiquées dans des protocoles connus.

19. Réseau selon l'une ou plusieurs des revendications précédentes, **caractérisé en ce qu'**il comprend une unité d'exploitation supplémentaire constitué d'un dispositif d'identification personnelle (10) du patient comprenant une mémoire dans laquelle au moins un code d'identification du patient est stocké et des moyens pour la lecture et l'écriture de ladite mémoire, ainsi que des moyens de communication avec d'autres unités d'exploitation à distance lors de la transmission et/ou de la réception.

20. Réseau selon la revendication 21, **caractérisé en ce que**, en plus du code d'identification du patient, la mémoire contient le type d'opération diagnostique et/ou thérapeutique à effectuer sur le patient.

21. Réseau selon la revendication 21 ou 22, **caractérisé en ce que** la mémoire contient des données sur les tests diagnostiques précédents et/ou sur les traitements thérapeutiques du patient, ainsi que sur les conditions pathologiques particulières du patient, lesdites données étant lues par les une ou plusieurs unités d'exploitation (3, 3', 6, 7, 8, 9) à l'intérieur du réseau, le réseau étant conçu pour vérifier la correspondance du médicament ou du produit de contraste utilisé et des paramètres d'utilisation et des protocoles de distribution avec celles prédéterminées pour les opérations diagnostiques et/ou thérapeutiques à effectuer, ainsi qu'à vérifier le bon choix du type d'opération diagnostique et/ou thérapeutique à effectuer sur le patient à l'examen avec le code d'identification stocké dans le dispositif d'identification (10).

22. Réseau selon l'une ou plusieurs des revendications précédentes, **caractérisé en ce qu'**il est conçu pour transmettre au serveur distant de gestion de la base de données des protocoles des médicaments, pour l'intégration de ladite base de données, des informations relatives au protocole employé, de façon manuelle et définie automatiquement et/ou des informations personnelles du patient et/ou des informations relatives à l'histoire médicale et à la pathologie du patient et/ou des informations relatives aux médicaments utilisés et aux modes de distribution et/ou des informations relatives à l'activité diagnostique et/ou thérapeutique et/ou chirurgicale, aussi bien que les résultats de la ou lesdites activités et/ou des informations relatives au mode de traitement des donnés diagnostiques détectés et/ou des informations relatives aux personnel qui utilise l'une ou plusieurs desdites unités.
